# EUROPEAN PATENT SPECIFICATION

(11) **EP 2 821 784 B1**
(45) Date of publication and mention of the grant of the patent: **13.11.2019**
(21) Application number: 12870268.5
(22) Date of filing: 20.07.2012
(51) Int. Cl.: G01N 33/00

(54) **METHOD FOR TREATING SURFACE OF RELEASING CHAMBER IN CONTACT WITH TEST OBJECT**
VERFAHREN ZUR OBERFLÄCHENBEHANDLUNG EINER FREISETZUNGSKAMMER IN KONTAKT MIT EINEM PRÜFGUT
PROCÉDÉ DE TRAITEMENT DE LA SURFACE D'UNE CHAMBRE DE LIBÉRATION EN CONTACT AVEC UN OBJET D'ÉTUDE

(30) Priority: 29.02.2012 CN 201210049393
(43) Date of publication of application: 07.01.2015
(73) Proprietor: Dongguan City Simplewell Technology Co., Ltd., Dongguan, Guangdong 523000 (CN)
(72) Inventor: XIA, Keyu, Dongguan Guangdong 523000 (CN)
(74) Representative: Cabinet Chaillot
(86) International application number: PCT/CN2012/078931
(87) International publication number: WO 2013/127149

(56) References cited:
- WO-A1-2011/081653
- CN-A- 1 360 644
- CN-U- 202 502 083
- DD-A1- 216 257
- US-A- 4 376 641
- DR.WENHAO CHEN: "STANDARD METHOD FOR THE TESTING AND EVALUATION OF VOLATILE ORGANIC CHEMICAL EMISSIONS FROM INDOOR SOURCES USING ENVIRONMENTAL CHAMBERS VERSION 1.1", INDOOR AIR QUALITY SECTION ENVIRONMENTAL HEALTH LABORATORY BRANCH DIVISION OF ENVIRONMENTAL AND OCCUPATIONAL DISEASE CONTROL CALIFORNIA DEPARTMENT OF PUBLIC HEALTH, 1 February 2010 (2010-02-01), pages 1-52, XP002744761,

## Description

### Technical Field

The present invention relates to a method for treating the surface of a test device for test of volatile organic compounds (VOC), semi-volatile organic compounds (SVOC) and high-boiling-point organic compounds in contact with a test object.

### Background Art

A traditional test device for test of volatile organic compounds (VOC), semi-volatile organic compounds (SVOC) and high-boiling-point organic compounds, such as a releasing chamber (also called a sampler), is composed of the following components: a chamber; a chamber door, a suction tube, an exhaust tube and a sampling tube connected with the chamber; a stirring fan mounted in the chamber; an air duct board (8); and other components; the inner wall of the chamber and the surface in contact with the test object of each component have some chemical bonds adsorbing the test object or strong surface tension, which results in the adsorption of the test object on the inner wall of the chamber and on the surface in contact with the test object of each component and hard removal therefrom. For polar compounds, the adsorption capacity is stronger due to the influence of some chemical bonds on the inner wall of the chamber and on the surface in contact with the test object of each component. Therefore, the inner wall of the chamber and the surface in contact with the test object of each component have to be treated, so as to make the chemical bonds on the inner wall of the test device such as a hydrogen bond, a silanol group and a Lewis acid activation point covered, which results in easy adsorption of the test object on the inner wall of the chamber and on the surface in contact with the test object of each component; when sampling for analysis of the content of the test object, a sample obtained from a sampling tube of the test device is used for the test; since the inner wall of the chamber and the surface in contact with the test object of each component adsorb a great amount of the test object, the tested value of the samples is less than the actual value of the test object in the chamber, having a great testing error, not effectively meeting the needs of a high-precision test.

Within the framework of the publication of Dr. Wenhao Chen: "Standard method for the testing and evaluation of volatile organic chemical emissions from indoor sources using environmental chambers, version 1.1", published by Indoor air quality section, environmental health laboratory branch, division of environmental and occupational disease control, California department of public health in February 2010, a standard test method for VOC testing using VOC emissions test chambers with electro-polished interior surfaces is disclosed. The described test chambers comprise a chamber inlet and exhaust which are positioned to ensure complete mixing of chamber air. The lid and door have a non-contaminating, non-sorbing gasket and an airtight seal mechanism.

Document DD 216257 A1 discloses the surface treatment of an environmental test chamber whereby a nickel coating is applied to the interior surface of the components of the test chamber, consisting of electrolessly coating Ni, stopping the process to produce an intermediate electroless coating of Cu.

### Disclosure of the Invention

The purpose of the present invention is to overcome the above defects of the prior art, and provide a method for treating the surface of a releasing chamber in contact with a test object, which can greatly reduce the adsorption of a test object on the inner wall of the chamber and on the surface in contact with the test object of each component, thus effectively improving the testing precision of the releasing chamber.

In order to achieve the above purpose, the present invention provides the following technical solution: A method is provided for treating the surface of a releasing chamber in contact with a test object, the releasing chamber comprising the following components: a chamber; a chamber door, a suction tube, an exhaust tube and a sampling tube connected with the chamber; a stirring fan mounted in the chamber; and an air duct board; the method is used for treating the surface of at least one component of the chamber, the chamber door, the suction tube, the exhaust tube, the sampling tube, the stirring fan and the air duct board in contact with the test object, comprising the following steps:
(1) with regard to the components made by processing stainless steel or glass, first washing each of the components to remove contaminants; if the component is stainless steel, the stainless steel component is oxidized with an acid and then washed with an organic solvent and water, or electrolyzed after oxidation and then washed with an organic solvent and water; if the component is glass, the glass component is corroded by HCL or HF, or the surface thereof is roughened by a physical method; other methods can also be used to wash each of the components to remove contaminants; and
(2) then follows production of a deactivated layer by processing the surface of the component washed in step (1), or first production of an intermediate layer by processing the surface of the component washed in step (1), and then production of a deactivated layer by processing the surface of the intermediate layer.

If the component is stainless steel, the SiO2 layer of said intermediate layer is obtained by calcining the component washed in step (1) at a temperature above 500°C with the introduction of monosilane, or by calcining the component washed in step (1) at a temperature above 500°C after coating, dip-coating or plating it with silicone, or by calcining the component washed in step (1) at a temperature above 500°C after coating, dip-coating or plating it with polysiloxanes and cyclodextrin derivatives, said polysiloxanes including a polydimethylsiloxane, a phenyl-containing polysiloxane, a cyanogen-containing polysiloxane, a fluorine-containing polysiloxane, a vinyl-containing polysiloxane, a hydrocarbyl-ended polysiloxane, or a polysiloxane with a space group introduced between a molecular chain and a functional group; said intermediate layer or epoxy compound and amine compound containing in a pore-forming agent no carbon atom or heterocycle derived from aromatic compounds undergo a polymerization reaction at 60-200°C to form a gelatinous substance; then coating or dipping the component washed in step (1), washing away the pore-forming agent with a solvent, and drying after leaving a skeleton phase to form a three-dimensional mesh or porous skeleton phase, said pore-forming agent including methyl cellosolve, ethyl cellosolve, methyl glycol acetate, propylene glycol monomethyl ether acetate and other esters, and polyglycol or polypropylene glycol, said epoxy compound including 2,2,2-tri-(2,3-epoxypropyl)-isocyanurate, said amine compound including ethanediamine, diethylene triamine, trithylenetetramine, tetraethylenepentamine, iminobispropylamine/dihexylenetriamine, 1,3,6-triaminomethylhexane, polymethylene diamine, trimethyl hexamethylene diamine, polyether diamine, isophorone diamine, menthane diamine, N-aminoethylpiperazine, 3,9-bis(3-aminopropyl)-2,4,8,10-tetraoxaspiro ring, bis(4-aminocyclohexyl)methane, or aliphatic polyamides made from polyamines and dimer acid; if the component is glass, the intermediate layer is produced by depositing silica, sodium chloride or carbon black onto the surface of the component.

Said deactivated layer is obtained by coating, dip-coating or plating the intermediate layer or the surface of the component with a low-surface-tension organic compound and wherein said low-surface-tension organic compound includes a fluorinated organic compound, a silane compound, hydrogen-containing silicone oil or poly-glycol, then baking it at a high temperature above 300°C; or said deactivated layer is obtained by coating, dip-coating or plating the surface of the component washed in step (1) or the intermediate layer with SiO2 sol and then baking it at a high temperature above 300°C and finally removing excess materials with a solvent; or said deactivated layer is obtained by coating, dip-coating or plating the surface of the component washed in step (1) or the intermediate layer with polybenzimidazole pyrrolidone (PY), polytetrafluoroethylene, polyfluoroalkyls, orthosilicate or ethyl orthosilicate and then baking it at a high temperature above 300°C, and whereby said intermediate layer (9) is a SiO₂ layer or in the form of a three-dimensional mesh or porous skeleton phase, having a thickness from submicron to micron.

The method of the present invention for treating the surface of a releasing chamber in contact with a test object has the following beneficial effects: The method is used for production of a deactivated layer by processing the surface of the component washed in step (1), or first production of an intermediate layer by processing the surface of the component washed in step (1), and then production of a deactivated layer by processing the surface of the intermediate layer. This reduces the surface tension of the inner wall of the chamber and the surface in contact with the test object of each component, which can greatly reduce the adsorption of a test object on the inner wall of the chamber and on the surface in contact with the test object of each component, so that the tested value of the samples taken from a sampling tube is closer to the actual value of the test object in the chamber, thus effectively improving the testing precision of the releasing chamber.

The method of the present invention for treating the surface of a releasing chamber in contact with a test object will be further described below with reference to drawings and examples.

### Brief Description of Drawings

Fig. 1 is a structural schematic drawing of the intermediate layer and deactivated layer applied to the releasing chamber as described in the method of the present invention for treating the surface of the releasing chamber in contact with a test object;
Fig. 2 is an enlarged sectional view along the line A-A in Fig. 1;
Fig. 3 is an enlarged sectional view along the line B-B in Fig. 1;
Fig. 4 is a structural schematic drawing of the coating layer applied to the releasing chamber as described in the method of the present invention for treating the surface of the releasing chamber in contact with a test object;
Fig. 5 is an enlarged sectional view along the line C-C in Fig. 4;
Fig. 6 is an enlarged sectional view along the line D-D in Fig. 4.

### Best Mode for Carrying out the Invention

The most preferred examples of the method of the present invention for treating the surface of a releasing chamber in contact with a test object are described as follows, but not thereby limit the scope of protection of the present invention.

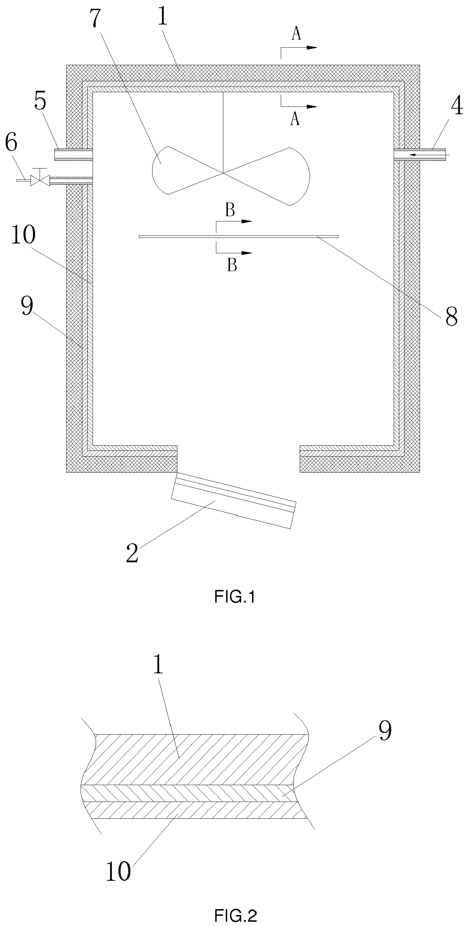

Example 1: As shown in Figs. 1-3, a method is provided for treating the surface of a releasing chamber in contact with a test object, the releasing chamber comprising the following components: a chamber 1; a chamber door 2, a suction tube 4, an exhaust tube 5 and a sampling tube 6 connected with the chamber 1; a stirring fan 7 mounted in the chamber 1; and an air duct board 8; the method is used for treating the surface of at least one component of the chamber 1, the chamber door 2, the suction tube 4, the exhaust tube 5, the sampling tube 6, the stirring fan 7 and the air duct board 8 in contact with the test object, comprising the following steps:
(1) with regard to the components made by processing stainless steel or glass, first cleaning each of the components to remove contaminants; if the component is stainless steel, the stainless steel component is oxidized with an acid and then washed with an organic solvent and water, or electrolyzed after oxidation and then washed with an organic solvent and water; if the component is glass, the glass component is corroded by HCL or HF, or the surface thereof is roughened by a physical method; other methods can also be used to wash each of the components to remove contaminants;
(2) first follows production of an intermediate layer 9 by processing the surface of the component washed in step (1), and then production of the deactivated layer 10 by processing the surface of the intermediate layer 9.

Said intermediate layer 9 is a SiO2 layer, or in the form of a three-dimensional mesh or porous skeleton phase, having a thickness from submicron to micron.

Said deactivated layer 10 has a thickness from submicron to micron and a liquid-crystal membrane structure.

If the component is stainless steel, the SiO2 layer of said intermediate layer 9 is obtained by calcining the component washed in step (1) at a temperature above 500°C with the introduction of monosilane, or by calcining the component washed in step (1) at a temperature above 500°C after coating, dip-coating or plating it with silicone, or by calcining the component washed in step (1) at a temperature above 500°C after coating, dip-coating or plating it with polysiloxanes and cyclodextrin derivatives, said polysiloxanes including a polydimethylsiloxane, a phenyl-containing polysiloxane, a cyanogen-containing polysiloxane, a fluorine-containing polysiloxane, a vinyl-containing polysiloxane, a hydrocarbyl-ended polysiloxane, or a polysiloxane with a space group introduced between a molecular chain and a functional group; said intermediate layer 9 or epoxy compound and amine compound containing in a pore-forming agent no carbon atom or heterocycle derived from aromatic compounds undergo a polymerization reaction at 60-200°C to form a gelatinous substance; then coating or dipping the component washed in step (1), washing away the pore-forming agent with a solvent, and drying after leaving a skeleton phase to form a three-dimensional mesh or porous skeleton phase, said pore-forming agent including methyl cellosolve, ethyl cellosolve, methyl glycol acetate, propylene glycol monomethyl ether acetate and other esters, and polyglycol or polypropylene glycol, said epoxy compound including 2,2,2-tri-(2,3-epoxypropyl)-isocyanurate, said amine compound including ethanediamine, diethylene triamine, trithylenetetramine, tetraethylenepentamine, iminobispropylamine/dihexylenetriamine, 1,3,6-triaminomethylhexane, polymethylene diamine, trimethyl hexamethylene diamine, polyether diamine, isophorone diamine, menthane diamine, N-aminoethylpiperazine, 3,9-bis(3-aminopropyl)-2,4,8,10-tetraoxaspiro ring, bis(4-aminocyclohexyl)methane, or aliphatic polyamides made from polyamines and dimer acid; if the component is glass, the intermediate layer 9 is produced by depositing silica, sodium chloride or carbon black onto the surface of the component.

Said deactivated layer 10 is obtained by coating, dip-coating or plating the intermediate layer 9 or the surface of the component with a low-surface-tension organic compound and then baking it at a high temperature above 300°C; or said deactivated layer 10 is obtained by coating, dip-coating or plating the surface of the component washed in step (1) or the intermediate layer 9 with SiO2 sol and then baking it at a high temperature above 300°C and finally removing excess materials with a solvent; or said deactivated layer 10 is obtained by coating, dip-coating or plating the surface of the component washed in step (1) or the intermediate layer 9 with polybenzimidazole pyrrolidone (PY), polytetrafluoroethylene, polyfluoroalkyls, orthosilicate or ethyl orthosilicate and then baking it at a high temperature above 300°C.

Said low-surface-tension organic compound includes a silane compound, hydrogen-containing silicone oil or polyglycol. Said silane compound includes chlorotrimethylsilane, hexamethyl disilazane, hydrocarbyl-ended polymethylsiloxane, phenyl-dimethyl polysilane, methyl trioxysilane, dimethyl polysiloxane, diphenyl tetramethyl silazane, polysiloxane or fluorine-containing polysiloxane.

Said low-surface-tension organic compound can also include a fluorinated organic compound.

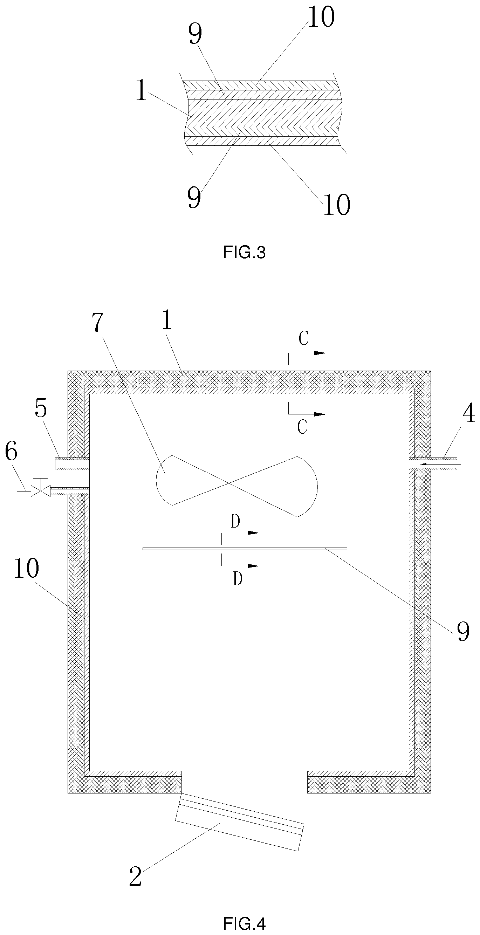

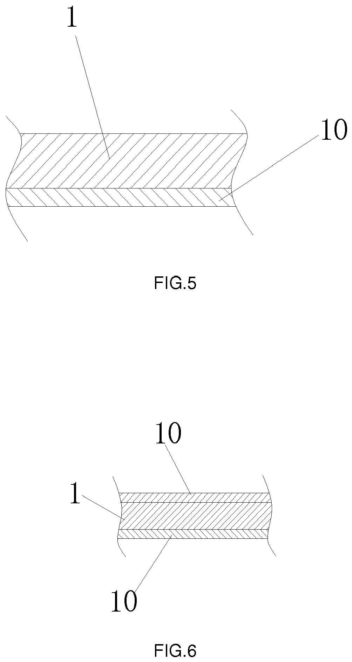

Example 2: As shown in Figs. 4-6, a method is provided for treating the surface of a releasing chamber in contact with a test object, the releasing chamber comprising the following components: a chamber 1; a chamber door 2, a suction tube 4, an exhaust tube 5 and a sampling tube 6 connected with the chamber 1; a stirring fan 7 mounted in the chamber 1; and an air duct board 8; the method is used for treating the surface of at least one component of the chamber 1, the chamber door 2, the suction tube 4, the exhaust tube 5, the sampling tube 6, the stirring fan 7 and the air duct board 8 in contact with the test object, comprising the following steps:
(1) with regard to the components made by processing stainless steel or glass, if the component is stainless steel, the stainless steel component is oxidized with an acid and then washed with an organic solvent and water, or electrolyzed after oxidation and then washed with an organic solvent and water; if the component is glass, and then washed with an organic solvent and water; and
(2) then follows direct production of a deactivated layer 10 by processing the surface of the component washed in step (1).

Said deactivated layer 10 has a thickness from submicron to micron and a liquid-crystal membrane structure.

Said deactivated layer 10 is obtained by coating, dip-coating or plating the intermediate layer 9 or the surface of the component with a low-surface-tension organic compound and then baking it at a high temperature above 300°C; or said deactivated layer 10 is obtained by coating, dip-coating or plating the surface of the component washed in step (1) or the intermediate layer 9 with SiO2 sol and then baking it at a high temperature above 300°C and finally removing excess materials with a solvent; or said deactivated layer 10 is obtained by coating, dip-coating or plating the surface of the component washed in step (1) or the intermediate layer 9 with polybenzimidazole pyrrolidone (PY), polytetrafluoroethylene, polyfluoroalkyls, orthosilicate or ethyl orthosilicate and then baking it at a high temperature above 300°C.

Said low-surface-tension organic compound includes a silane compound, hydrogen-containing silicone oil or polyglycol. Said silane compound includes chlorotrimethylsilane, hexamethyl disilazane, hydrocarbyl-ended polymethylsiloxane, phenyl-dimethyl polysilane, methyl trioxysilane, dimethyl polysiloxane, diphenyl tetramethyl silazane, polysiloxane or fluorine-containing polysiloxane.

Said low-surface-tension organic compound can also include a fluorinated organic compound.

The above examples are preferred embodiments of the present invention, but the embodiments of the present invention are free from restriction of the above examples, and any other alteration, modification, substitution, combination and simplification made without departing from the spiritual essence and principle of the present invention are equivalent replacements and fall within the scope of protection of the present invention.

## Claims

1. A method for treating a surface of a releasing chamber in contact with a test object, the releasing chamber comprising the following components: a chamber (1); a chamber door (2), a suction tube (4), an exhaust tube (5) and a sampling tube (6) connected with the chamber (1); a stirring fan (7) mounted in the chamber (1); and an air duct board (8); the method is used for treating the surface of at least one component of the chamber (1), the chamber door (2), the suction tube (4), the exhaust tube (5), the sampling tube (6), the stirring fan (7) and the air duct board (8) in contact with the test object, **characterized in that** the following steps are comprised:
(1) washing the components of the releasing chamber made by processing stainless steel or glass, to remove contaminants; and
(2) production of a deactivated layer (10) by processing the surface of the component washed in step (1), or first production of an intermediate layer (9) by processing the surface of the component washed in step (1), and then production of the deactivated layer (10) by processing the surface of the intermediate layer (9), whereby the deactivated layer (10) is obtained by coating, dip-coating or plating the intermediate layer (9) or the surface of the component washed in step (1) with a low-surface-tension organic compound and wherein the low-surface-tension organic compound includes a fluorinated organic compound, a silane compound, hydrogen-containing silicone oil or poly-glycol, and then baking it at a high temperature above 300°C or whereby said deactivated layer (10) is obtained by coating, dip-coating or plating the surface of the component washed in step (1) or the intermediate layer (9) with SiO₂ sol and then baking it at a high temperature above 300 °C and finally removing excess materials with a solvent or whereby said deactivated layer (10) is obtained by coating, dip-coating or plating the surface of the component washed in step (1) or the intermediate layer (9) with polybenzimidazole pyrrolidone (PY), polytetrafluoroethylene, polyfluoroalkyls, orthosilicate or ethyl orthosilicate and then baking it at a high temperature above 300°C, and whereby said intermediate layer (9) is a SiO₂ layer or in the form of a three-dimensional mesh or porous skeleton phase, having a thickness from submicron to micron.

2. The method for treating the surface of the releasing chamber in contact with the test object according to claim 1, **characterized in that**, with regard to the components made by processing stainless steel or glass, if the component is stainless steel, the stainless steel component is oxidized with an acid and then washed with an organic solvent and water, or electrolyzed after oxidation and then washed with an organic solvent and water; if the component is glass, the glass component is corroded by HCL or HF, or the surface thereof is roughened by a physical method.

3. The method for treating the surface of the releasing chamber in contact with the test object according to claim 1, **characterized in that** said deactivated layer (10) has a thickness from submicron to micron and a liquid-crystal membrane structure.

4. The method for treating the surface of the releasing chamber in contact with the test object according to claim 1, **characterized in that** if the component is stainless steel, the SiO₂ layer of said intermediate layer (9) is obtained by calcining the component washed in step (1) at a temperature above 500 with the introduction of monosilane, or by calcining the component washed in step (1) at a temperature above 500°C after coating, dip-coating or plating it with silicone, or by calcining the component washed in step (1) at a temperature above 500°C after coating, dip-coating or plating it with polysiloxanes and cyclodextrin derivatives, said polysiloxanes including a polydimethylsiloxane, a phenyl-containing polysiloxane, a cyanogen-containing polysiloxane, a fluorine-containing polysiloxane, a vinyl-containing polysiloxane, a hydrocarbyl-ended polysiloxane, or a polysiloxane with a space group introduced between a molecular chain and a functional group; said intermediate layer (9) or epoxy compound and amine compound containing in a pore-forming agent no carbon atom or heterocycle derived from aromatic compounds undergo a polymerization reaction at 60-200°C to form a gelatinous substance; then coating or dipping the component washed in step (1), washing away the pore-forming agent with a solvent, and drying after leaving a skeleton phase to form a three-dimensional mesh or porous skeleton phase, said pore-forming agent including methyl cellosolve, ethyl cellosolve, methyl glycol acetate, propylene glycol monomethyl ether acetate and other esters, and polyglycol or polypropylene glycol, said epoxy compound including 2,2,2-tri-(2,3-epoxypropyl)-isocyanurate, said amine compound including ethanediamine, diethylene triamine, trithylenetetramine, tetraethylenepentamine, iminobispropylamine/dihexylenetriamine, 1,3,6-triaminomethylhexane, polymethylene diamine, trimethyl hexamethylene diamine, polyether diamine, isophorone diamine, menthane diamine, N-aminoethylpiperazine, 3,9-bis(3-aminopropyl)-2,4,8,10-tetraoxaspiro ring, bis(4-aminocyclohexyl)methane, or aliphatic polyamides made from polyamines and dimer acid; if the component is glass, the intermediate layer (9) is produced by depositing silica, sodium chloride or carbon black onto the surface of the component.

5. The method for treating the surface of the releasing chamber in contact with the test object according to claim 1, **characterized in that** said SiO₂ sol is composed of methyltriethoxysilane and tetraethoxysilane hydrolyzate.

6. The method for treating the surface of the releasing chamber in contact with the test object according to claim 1, **characterized in that** said silane compound includes chlorotrimethylsilane, hexamethyl disilazane, hydrocarbyl-ended polymethylsiloxane, phenyl-dimethyl polysilane, methyl trioxysilane, dimethyl polysiloxane, diphenyl tetramethyl silazane, polysiloxane or fluorine-containing polysiloxane.

## Patentansprüche

1. Verfahren zum Bearbeiten einer Oberfläche einer Freisetzungskammer, die mit einem Testobjekt in Kontakt ist, wobei die Freisetzungskammer die folgenden Bauteile umfasst: eine Kammer (1); eine Kammertür (2), ein Ansaugrohr (4), ein Absaugrohr (5) und ein Beprobungsrohr (6), die an die Kammer (1) angeschlossen sind; einen Rührlüfter (7), der in der Kammer (1) montiert wird; und eine Luftkanal-Platte (8); das Verfahren ist zum Bearbeiten der Oberfläche wenigstens eines Bauteils der Kammer (1), der Kammertür (2), des Ansaugrohrs (4), des Absaugrohrs (5), des Beprobungsrohr (6), des Rührlüfters (7) und der Luftkanal-Platte (8), der mit dem Testobjekt in Kontakt ist, verwendet, **dadurch gekennzeichnet, dass** die folgenden Schritte umfasst sind:
(1) Waschen der Bauteile der Freisetzungskammer, die durch Verarbeiten von Edelstahl oder Glas hergestellt sind, um Kontaminanten zu entfernen; und
(2) Erzeugen einer deaktivierten Schicht (10) durch Verarbeiten der Oberfläche des in Schritt (1) gewaschenen Bauteils oder erstes Erzeugen einer Zwischenschicht (9) durch Verarbeiten der Oberfläche des in Schritt (1) gewaschenen Bauteils und dann Erzeugen der deaktivierten Schicht (10) durch Verarbeiten der Oberfläche der Zwischenschicht (9), wodurch die deaktivierte Schicht (10) durch Beschichten, Tauchlackieren oder Plattieren der Zwischenschicht (9) oder der Oberfläche des in Schritt (1) gewaschenen Bauteils mit einer organischen Verbindung mit niedriger Oberflächenspannung erhalten ist und wobei die organische Verbindung mit niedriger Oberflächenspannung eine fluorierte organische Verbindung, eine Silan-Verbindung, ein Wasserstoff enthaltendes Silikonöl oder Poly-Glykol einschließt, und dann Backen bei einer hohen Temperatur von über 300° C oder wodurch die genannte deaktivierte Schicht (10) durch Beschichten, Tauchlackieren oder Plattieren der Oberfläche des in Schritt (1) gewaschenen Bauteils oder die Zwischenschicht (9) mit SiO₂-Sol erhalten ist und dann Backen bei einer hohen Temperatur über 300° C und schließlich Entfernen des überschüssigen Materials mit einem Lösungsmittel oder wodurch die genannte deaktivierte Schicht (10) durch Beschichten, Tauchlackieren oder Plattieren der Oberfläche des in Schritt (1) gewaschenen Bauteils oder die Zwischenschicht (9) mit Polybenzimidazol-Pyrrolidon (PY), Polytetrafluorethylen, Polyfluoralkyl, Orthosilicat oder Ethyl-Orthosilicat erhalten ist, und dann Backen bei einer hohen Temperatur über 300° C, und wodurch die genannte Zwischenschicht (9) eine SiO₂-Schicht oder in der Form eines dreidimensionalen Netzes oder einer porösen Skelettphase mit einer Dicke von Submikronen oder Mikronen ist.

2. Verfahren zum Bearbeiten der Oberfläche der Freisetzungskammer, die mit einem Testobjekt in Kontakt ist, gemäß Anspruch 1, **dadurch gekennzeichnet, dass** in Bezug auf die Bauteile, die durch Verarbeiten von Edelstahl oder Glas hergestellt sind, wenn das Bauteil Edelstahl ist, das Edelstahl-Bauteil mit einer Säure oxidiert ist und dann mit einem organischen Lösungsmittel und Wasser gewaschen wird oder nach dem Oxidieren elektrolysiert und dann mit einem organischen Lösungsmittel und Wasser gewaschen wird; wenn das Bauteil Glas ist, wird das Glasbauteil durch HCl oder HF korrodiert oder dessen Oberfläche wird durch ein physikalisches Verfahren aufgeraut.

3. Verfahren zum Bearbeiten der Oberfläche der Freisetzungskammer, die mit einem Testobjekt in Kontakt ist, gemäß Anspruch 1, **dadurch gekennzeichnet, dass** die genannte deaktivierte Schicht (10) eine Dicke von Submikronen bis Mikronen und eine Flüssigkristall-Membranstruktur aufweist.

4. Verfahren zum Bearbeiten der Oberfläche der Freisetzungskammer, die mit dem Testobjekt in Kontakt ist, gemäß Anspruch 1, **dadurch gekennzeichnet, dass**, wenn das Bauteil Edelstahl ist, die SiO₂-Schicht der genannten Zwischenschicht (9) durch Glühen des in Schritt (1) gewaschenen Bauteils bei einer Temperatur von über 500 mit dem Einführen von Monosilan oder durch Glühen des in Schritt (1) gewaschenen Bauteils bei einer Temperatur von über 500° C nach dem Beschichten, Tauchlackieren oder Plattieren mit Silikon oder durch Glühen des in Schritt (1) gewaschenen Bauteils bei einer Temperatur von über 500° C, nach dem Beschichten, Tauchlackieren oder Plattieren mit Polysiloxanen und Cyclodextrin-Derivaten erhalten wird, wobei die genannten Polysiloxane ein Polydimethylsiloxan, ein Phenyl enthaltendes Polysiloxan, ein Zyan enthaltendes Polysiloxan, ein Fluor enthaltendes Polysiloxan, ein Vinyl enthaltendes Polysiloxan, ein Polysiloxan mit einem Hydrocarbyl-Ende oder ein Polysiloxan mit einer Raumgruppe einschließen, die zwischen einer Molekularkette und einer funktionalen Gruppe eingeführt ist; wobei die genannte Zwischenschicht (9) oder die Epoxid-Verbindung und die Amin-Verbindung in einem porenbildenden Wirkstoff kein Kohlenstoffatom oder keinen Heterozyklus enthält, der von aromatischen Verbindungen abgeleitet ist, einer Polymerisationsreaktion bei 60 - 200° C unterzogen werden, um eine gelatinöse Substanz zu bilden; dann Beschichten oder Eintauchen des in Schritt (1) gewaschenen Bauteils, Wegwaschen des porenbildenden Wirkstoffs mit einem Lösungsmittel und Trocknen, nachdem eine Skelettphase zum Bilden eines dreidimensionalen Netzes oder einer porösen Skelettphase gelassen wird, wobei der genannte porenbildende Wirkstoff Methyl-Cellose, Ethyl-Cellulose Methylglykol-Acetat, Propylenglykol-Monomethylether-Acetat und andere Ester und Polyglykol oder Polypropylen-Glykol einschließt, wobei die genannte Epoxy-Verbindung 2,2,2-tri-(2,3-Epoxypropyl)-Isocyanurat einschließt, wobei die genannte Amin-Verbindung Ethandiamin, Diethylentriamin, Triethylentetramin, Tetraethylenpentamin, Iminobispropylamin / Dihexylentriamin, 1,3,6-Triaminomethylhexan, Polymethylen-Diamin, Trimethylhexamethylen-Diamin, Polyether-Diamin, IsophoronDiamin, Menthan-Diamin, N-Aminoethylpiperazin, 3,9,-bis(3-Aminopropyl)-2,4,8,10-Tetraoxaspiro-Ring, bis(4-Aminocyclohexyl)Methan oder aliphatische Polyamide, die aus Polyaminen und Dimersäure hergestellt sind, einschließt; wenn das Bauteil Glas ist, wird die Zwischenschicht (9) durch Aufbringen von Siliziumoxid, Natriumchlorid oder Kohlenschwarz auf die Oberfläche des Bauteils erzeugt.

5. Verfahren zum Bearbeiten der Oberfläche der Freisetzungskammer, die mit dem Testobjekt in Kontakt ist, gemäß Anspruch 1, **dadurch gekennzeichnet, dass** das genannte SiO₂-Sol aus Methyltriethoxysilan und Tetraethoxysilan-Hydrolyzat zusammengesetzt ist.

6. Verfahren zum Bearbeiten der Oberfläche der Freisetzungskammer, die mit einem Testobjekt in Kontakt ist, gemäß Anspruch 1, **dadurch gekennzeichnet, dass** die genannte Silan-Verbindung Chlortrimethylsilan, HexamethylDisilazan, Polyethylsiloxan mit Hydrocarbyl-Ende, Phenyl-Dimethylpolysilan, Methyl-Trioxylsilan, DimethylPolysiloxan, Diphenyl-Tetramethyl-Silazan, Polysiloxan oder Fluor enthaltendes Polysiloxan einschließt.

## Revendications

1. Procédé de traitement d'une surface d'une chambre de libération en contact avec un objet de test, la chambre de libération comprenant les composants suivants : une chambre (1) ; une porte de chambre (2), un tube d'aspiration (4), un tube d'échappement (5) et un tube d'échantillonnage (6) reliés à la chambre (1) ; un ventilateur d'agitation (7) monté dans la chambre (1) ; et un panneau pour conduit d'air (8) ; le procédé est utilisé pour traiter la surface d'au moins un composant de la chambre (1), de la porte de chambre (2), du tube d'aspiration (4), du tube d'échappement (5), du tube d'échantillonnage (6), du ventilateur d'agitation (7) et du panneau pour conduit d'air (8) en contact avec l'objet de test, **caractérisé par le fait que** les étapes suivantes sont comprises :
(1) lavage des composants de la chambre de libération obtenus par traitement d'acier inoxydable ou de verre, pour éliminer les contaminants ; et
(2) production d'une couche désactivée (10) par traitement de la surface du composant lavé dans l'étape (1), ou d'abord production d'une couche intermédiaire (9) par traitement de la surface du composant lavé dans l'étape (1), puis production de la couche désactivée (10) par traitement de la surface de la couche intermédiaire (9), ce par quoi la couche désactivée (10) est obtenue par revêtement, revêtement par immersion ou placage de la couche intermédiaire (9) ou de la surface du composant lavé dans l'étape (1) par un composé organique à faible tension superficielle et où le composé organique à faible tension superficielle comprend un composé organique fluoré, un composé silane, une huile de silicone contenant de l'hydrogène ou un poly-glycol, puis cuisson à une température élevée au-dessus de 300°C ou ce par quoi ladite couche désactivée (10) est obtenue par revêtement, revêtement par immersion ou placage de la surface du composant lavé dans l'étape (1) ou de la couche intermédiaire (9) par un sol de SiO₂, puis cuisson à une température élevée au-dessus de 300°C et finalement retrait des matières en excès par un solvant ou ce par quoi ladite couche désactivée (10) est obtenue par revêtement, revêtement par immersion ou placage de la surface du composant lavé dans l'étape (1) ou de la couche intermédiaire (9) par de la polybenzimidazole pyrrolidone (PY), du polytétrafluoroéthylène, des polyfluoroalkyles, de l'orthosilicate ou de l'orthosilicate d'éthyle, puis cuisson à une température élevée au-dessus de 300°C, et ce par quoi ladite couche intermédiaire (9) est une couche de SiO₂ ou dans la forme d'une phase de maillage tridimensionnel ou de squelette poreux, ayant une épaisseur allant du sous-micron au micron.

2. Procédé de traitement de la surface de la chambre de libération en contact avec l'objet de test, selon la revendication 1, **caractérisé par le fait qu'**en ce qui concerne les composants obtenus par traitement de l'acier inoxydable ou du verre, si le composant est en acier inoxydable, le composant en acier inoxydable est oxydé par un acide puis lavé avec un solvant organique et de l'eau, ou électrolysé après oxydation puis lavé avec un solvant organique et de l'eau ; si le composant est en verre, le composant en verre est corrodé par HCL ou HF, ou la surface de celui-ci est rendue rugueuse par une méthode physique.

3. Procédé de traitement de la surface de la chambre de libération en contact avec l'objet test, selon la revendication 1, **caractérisé par le fait que** ladite couche désactivée (10) a une épaisseur allant du sous-micron au micron et une structure de membrane cristal liquide.

4. Procédé de traitement de la surface de la chambre de libération en contact avec l'objet test, selon la revendication 1, **caractérisé par le fait que**, si le composant est en acier inoxydable, la couche de SiO₂ de ladite couche intermédiaire (9) est obtenue par calcination du composant lavé dans l'étape (1) à une température au-dessus de 500°C avec l'introduction de monosilane, ou par calcination du composant lavé dans l'étape (1) à une température au-dessus de 500°C après revêtement, revêtement par immersion ou placage de celui-ci par du silicone, ou par calcination du composant lavé dans l'étape (1) à une température au-dessus de 500°C après revêtement, revêtement par immersion ou placage de celui-ci par des polysiloxanes et des dérivés de cyclodextrine, lesdits polysiloxanes comprenant un polydiméthylsiloxane, un polysiloxane à teneur en phényle, un polysiloxane à teneur en cyanogène, un polysiloxane à teneur en fluor, un polysiloxane à teneur en vinyle, un polysiloxane terminé par hydrocarbyle ou un polysiloxane avec un groupe espaceur introduit entre une chaîne moléculaire et un groupe fonctionnel ; ladite couche intermédiaire (9) ou composé époxy et composé amine contenant dans un agent porogène aucun atome de carbone ou hétérocycle dérivé de composés aromatiques subissent une réaction de polymérisation à 60-200°C pour former une substance gélatineuse ; puis revêtement ou immersion du composant lavé dans l'étape (1), retrait par lavage de l'agent porogène avec un solvant, et séchage après qu'on ait laissé une phase de squelette former une phase de maillage tridimensionnel ou de squelette poreux, ledit agent porogène comprenant du méthyl cellosolve, de l'éthyl cellosolve, du méthyl glycol acétate, du propylène glycol monométhyl éther acétate et autres esters, et du polyglycol ou du polypropylène glycol, ledit composé époxy comprenant du 2,2,2-tri-(2,3-époxypropyl)-isocyanurate, ledit composé amine comprenant de l'éthane diamine, de la diéthylène triamine, de la triéthylène tétramine, de la tétraéthylène pentamine, de l'iminobispropylamine/dihexylènetriamine, du 1,3,6-triaminométhylhexane, de la polyméthylène diamine, de la triméthyl hexaméthylène diamine, de la polyéther diamine, de l'isophorone diamine, de la menthane diamine, de la N-aminoéthylpipérazine, du 3,9-bis(3-aminopropyl)-2,4,8,10-tétraoxaspiro cycle, du bis(4-aminocyclohexyl)méthane, ou des polyamides aliphatiques obtenus à partir de polyamines et d'acide dimère ; si le composant est en verre, la couche intermédiaire (9) est produite par dépôt de silice, de chlorure de sodium ou de noir de carbone sur la surface du composant.

5. Procédé de traitement de la surface de la chambre de libération en contact avec l'objet de test, selon la revendication 1, **caractérisé par le fait que** ledit sol de SiO₂ est composé d'hydrolysat de méthyltriéthoxysilane et de tétraéthoxysilane.

6. Procédé de traitement de la surface de la chambre de libération en contact avec l'objet test, selon la revendication 1, **caractérisé par le fait que** ledit composé silane comprend le chlorotriméthyl silane, l'hexaméthyl disilazane, le polyméthyl siloxane terminé par hydrocarbyle, le phényl-diméthyl polysilane, le méthyl trioxysilane, le diméthyl polysiloxane, le diphényl tétraméthyl silazane, le polysiloxane ou le polysiloxane contenant du fluor.
